# EUROPEAN PATENT APPLICATION

(11) **EP 4 497 466 A1**
(43) Date of publication of application: **29.01.2025**
(21) Application number: 23773982.6
(22) Date of filing: 23.03.2023
(51) Int. Cl.: A61M 60/165, A61M 60/139

(54) **BLOOD PUMP SYSTEM**

(30) Priority: 23.03.2022 CN 202210316344
(71) Applicant: Shanghai Golden Leaf Med Tec Co., Ltd, Xuhui District Shanghai 200231 (CN)
(72) Inventor: JI, Liang, Shanghai 200231 (CN); CAO, Songyezi, Shanghai 200231 (CN); SHEN, Meijun, Shanghai 200231 (CN); CHEN, Weihua, Shanghai 200231 (CN); CHEN, Jiafeng, Shanghai 200231 (CN); CHAN, Genan, Shanghai 200231 (CN); CHEN, Jun, Shanghai 200231 (CN); SHAO, Wenbin, Shanghai 200231 (CN)
(74) Representative: Wang, Bo
(86) International application number: PCT/CN2023/083515
(87) International publication number: WO 2023/179737

(57) **Abstract**

A blood pump system, comprising: a heart circulation pump (1) for being arranged in a heart so as to pump blood in the heart into an aorta; an aorta circulation pump (2) for being arranged in the aorta so as to supply blood in the aorta to a set organ; and a power transmission section (3) connected between the heart circulation pump (1) and the aorta circulation pump (2) so as to realize power transmission. The blood pump system is capable of accelerating the circulation of the blood in the heart and the blood in the aorta by means of the heart circulation pump (1) and the aorta circulation pump (2), respectively, thus assisting in improving the blood supply function of the heart. Moreover, rotating blades (112) of the blood pump system are adjustable. When in use, the rotating blades in a fully folded state allow the blood pump system to be placed into a body by means of a simple interventional operation, and then the rotating blades are unfolded to a semi-unfolded state or a fully unfolded state in the body to improve the blood flow condition.

## Description

### BACKGROUND

### Technical Field

The disclosure relates to a blood pump system, particularly to a blood pump system using a dual-pump aortic circulation structure, belonging to the field of medical device technology.

### Related Art

Cardiac dysfunction, also known as heart failure, is accompanied by clinical symptoms of heart dysfunction. Heart failure is a group of syndromes with various cardiac structural or functional diseases leading to ventricular filling and (or) impaired ejection function, and cardiac output cannot meet the needs of body tissue metabolism, with pulmonary circulation and (or) systemic circulation congestion, and organ or tissue blood perfusion insufficiency as clinical manifestations. Hear failure is mainly manifested as dyspnea, physical activity limitation and body fluid retention. For patients with cardiac dysfunction, cardiac assist devices can be used to improve symptoms. However, current cardiac assist devices generally suffer from problems such as large size and complex surgical implantation into the body.

### SUMMARY

The technical problem to be solved by the present disclosure is to provide a blood pump system using a dual pump aortic circulation structure.

To achieve the above technical objectives, the present disclosure adopts the following technical solutions:
A blood pump system comprising:
a heart circulation pump, used to be set inside the heart to extract blood from the heart into the aorta;
an aortic circulation pump, used to be installed inside the aorta to supply blood to designated organs;
a power transmission section connected between the heart circulation pump and the aortic circulation pump to achieve power transmission.

Preferably, the heart circulation pump comprises:
a first rotating member, mounted on the power transmission section to rotate with the power transmission section;
a first mesh stent, installed on an outer side of the first rotating member to protect to the first rotating member; and
a first guiding tube, sleeved on the outer side of the first mesh stent and including a first blood inlet and a first blood outlet, wherein the first blood inlet is far away from the aortic circulation pump and the first blood outlet is close to the aortic circulation pump,
the power transmission section configured to drive the first rotating member to rotate, so as to drive blood in a heart to enter the first guiding tube from the first blood inlet of the first guiding tube and then flow out of the first guiding tube from the first blood outlet.

Preferably, the aortic circulation pump comprises:
a second rotating member, mounted on the power transmission section to rotate with the rotation of the power transmission section;
a second mesh stent, installed on an outer side of the second rotating member for protection to the second rotating member;
a supporting stent, being able to spread over the power transmission section in an open state to support an aorta; and
a second guiding tube, sleeved on an outer side of the second mesh stent and including a second blood inlet and a second blood outlet, wherein the second blood inlet is close to the first blood outlet and the second blood outlet is far away from the first blood outlet.

Preferably, the first rotating member comprises:
a rotating body, sleeved on the power transmission section to rotate with the rotation of the power transmission section, and being opened with a plurality of rotating shaft grooves for accommodating the rotating blades;
a plurality of rotating blades, which can be accommodated in and extend from the rotating shaft grooves to adjust an opening angle of the rotating blades relative to the rotating body.

Preferably, the first mesh stent is braided from a plurality of first metal wires, and a middle section of each first metal wire is bent into a trapezoidal shape, so that both ends of the first metal wires are sleeved on the power transmission section, and the middle section of the first metal wires forms a cylindrical cavity for accommodating the first rotating member.

Preferably, the supporting stent is braided from multiple second metal wires, and a first end of each second metal wire is close to the power transmission section, and a second end of each second metal wire is far away from the power transmission section, so that the first ends of the second metal wires are collectively sleeved on the power transmission section, and the second ends of the second metal wires are spread outwardly in a petal shape.

Preferably, the first mesh stent is laser engraved from a metal tube, so that both ends of the first mesh stent are fitted onto the power transmission section, and the middle section of the first mesh stent forms a cylindrical cavity for accommodating the first rotating member.

Preferably, the supporting stent is laser engraved from a metal tube, so that a first end of the supporting stent is sleeved on the power transmission section, and a second end of the supporting stent is spread outwardly in a petal shape.

Preferably, the power transmission section comprises a cardiac transmission section, an aortic transmission section, and a power cable;
the cardiac transmission section is directly connected to the power cable to control the rotation of the first rotating member through the power cable;
the aortic transmission section is directly connected to the power cable to control the rotation of the second rotating member through the power cable;
the cardiac transmission section is connected to the aortic transmission section, which is connected to the power cable to control the simultaneous rotation of the first rotating member and the second rotating member through the power cable.

Preferably, the first guiding tube comprises a first acceleration section and a second acceleration section, which are spaced along the length direction of the first guiding tube to increase flow velocity of blood inside the first guiding tube.

Preferably, the first acceleration section comprises a first arc-shaped tube and a second arc-shaped tube with gradually changing diameters, and both the first arc-shaped tube and the second arc-shaped tube are bent towards the axis direction of the first guiding tube;
the diameter of the first arc-shaped tube decreases, and the diameter of the second arc-shaped tube increases;
the bending radius of the first arc-shaped tube has a first set ratio to the bending radius of the second arc-shaped tube.

Preferably, the second acceleration section comprises a third arc-shaped tube and a fourth arc-shaped tube, both of which are bent towards the axis direction of the first guiding tube; the diameter of the third arc-shaped tube decreases, and the diameter of the fourth arc-shaped tube increases; the bending radius of the third arc-shaped tube has a second set ratio to the bending radius of the fourth arc-shaped tube.

Compared with the existing technology, the blood pump system provided in the embodiments of the present disclosure can accelerate the circulation of blood in the heart and the aorta respectively through the heart circulation pump and the aortic circulation pump, thereby assisting in improving the blood supply function of the heart. Moreover, the rotating blades of the blood pump system are adjustable. When in use, the blood pump system can be easily inserted into the body through a simple interventional surgery in a fully-folded state (with a smaller size at this state), and then opened to a semi-unfolded or fully-unfolded state in the body to increase blood flow and enhance convenience of use.

### BRIEF DESCRIPTION OF THE DRAWINGS

FIG. 1 is a schematic diagram of the overall structure of a blood pump system provided in the first embodiment of the present disclosure;
FIG. 2 is a schematic diagram of the exploded structure of the heart circulation pump in FIG. 1;
FIG. 3 is a schematic diagram of the structure of the first rotating member in FIG. 2;
FIG. 4 is a schematic diagram of the structure of the rotating blades of the first rotating member at different opening angles;
FIG. 5 is a schematic diagram of the structure of the blood pump system in different forms with different opening angles;
FIG. 6 is a schematic diagram of the structure of the first mesh stent;
FIG. 7 is a schematic diagram of the structure of the first guiding tube;
FIG. 8 is a schematic diagram of the disassembled structure of the aortic circulation pump;
FIG. 9 is a schematic diagram of the structure of the supporting stent;
FIG. 10 is a schematic diagram of the disassembled structure of the power transmission section;
FIG. 11 illustrates the actual application scenario of the heart circulation pump;
FIG. 12 illustrates the actual application scenario of the aortic circulation pump;
FIG. 13 is a schematic diagram of the structure of a blood pump system provided in a second embodiment of the present disclosure;
FIG. 14 is a schematic diagram of the structure of a blood pump system provided in a third embodiment of the present disclosure.

### DETAILED DESCRIPTION

The technical content of the present disclosure will be described in detail below in combination with the accompanying drawings and specific embodiments.

### First Embodiment

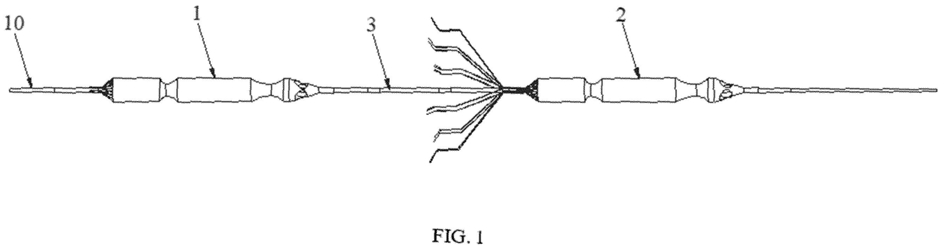

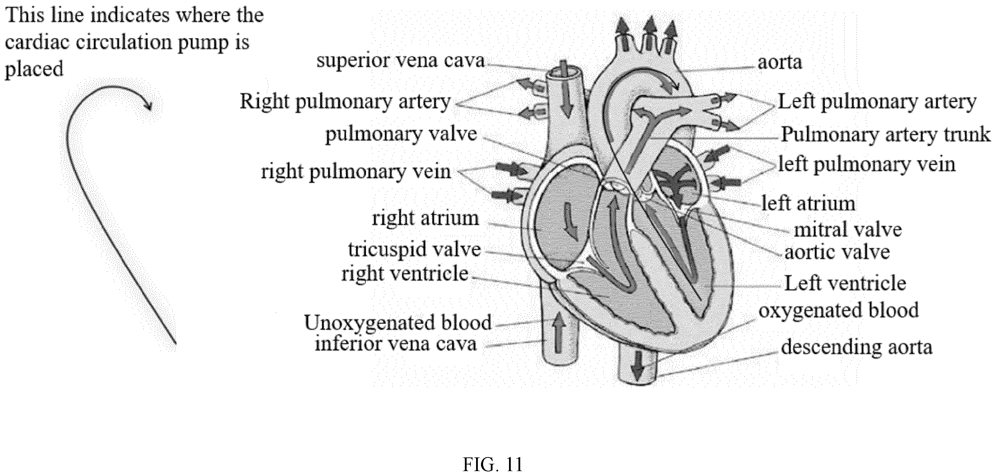

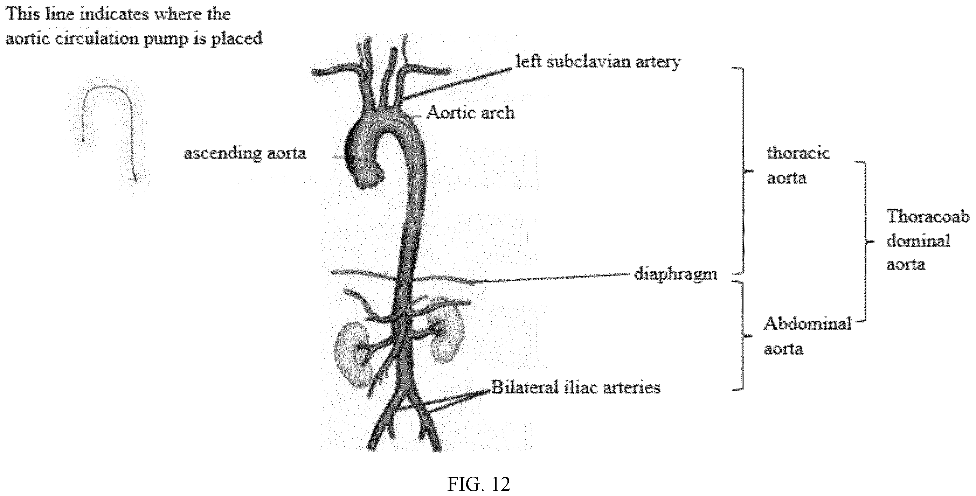

FIG.1 shows a blood pump system provided in the first embodiment of the present disclosure, which specifically includes a heart circulation pump 1, an aortic circulation pump 2, and a power transmission section 3. The heart circulation pump 1 is used to be located inside the heart (as shown in FIG. 11, in the present embodiment it is set inside the left ventricle, or it can be set inside the atrium), in order to extract blood from the heart into the aorta. Aortic circulation pump 2 is used to be installed inside the aorta (as shown in FIG. 12, in this embodiment, it is installed inside the descending aorta, or it can be installed inside the aortic arch), in order to supply blood from the aorta to the designated organs. The power transmission section 3 is connected between the heart circulation pump 1 and the aortic circulation pump 2 to achieve power transmission.

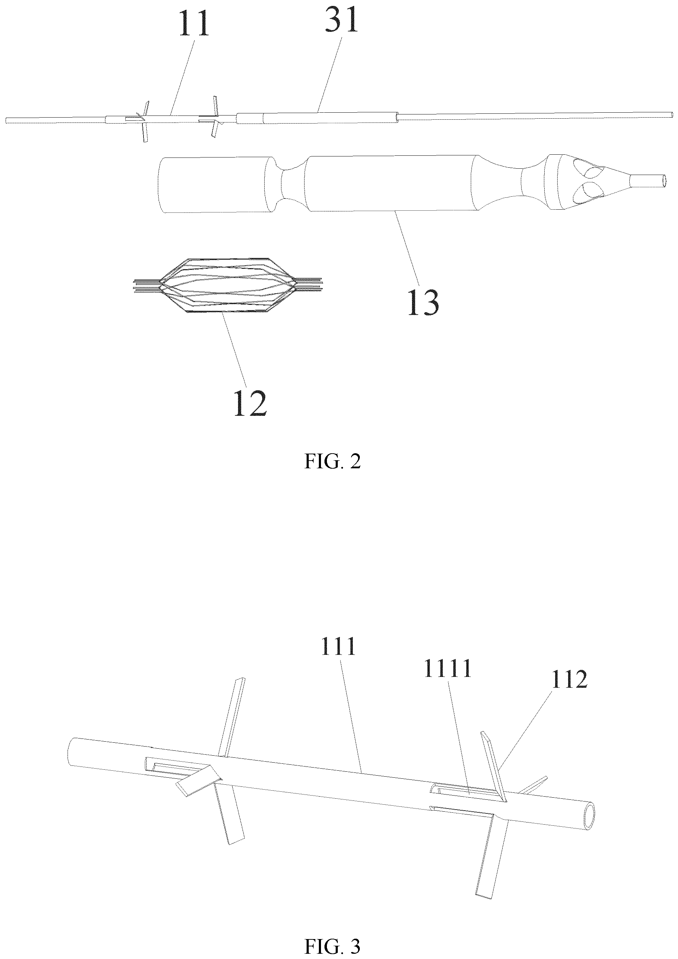

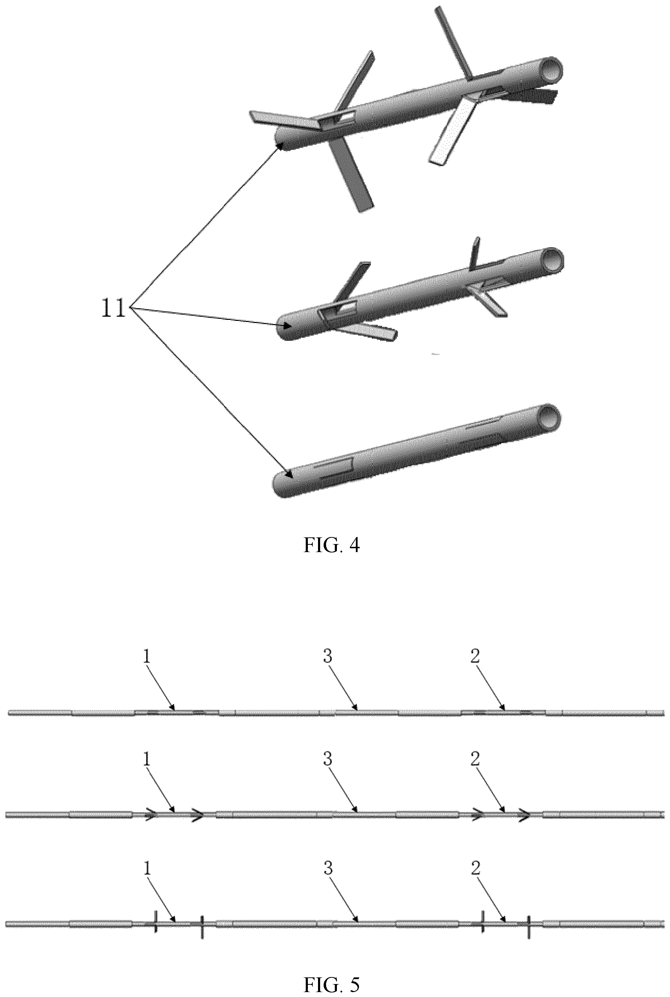

As shown in FIG. 2, in the present embodiment, the heart circulation pump 1 includes a first rotating member 11, a first mesh stent 12, and a first guide tube 13. Specifically, the first rotating member 11 is mounted on the power transmission section 3 to rotate together with the power transmission section 3. Moreover, the first rotating member 11 has a connection section 10 at an end far from the aortic circulation pump 2, for connecting with a pigtail elbow. As shown in FIG. 3, in one embodiment of the present disclosure, the first rotating member 11 includes a rotating body 111 and a plurality of rotating blades 112. The rotating body 111 is sleeved on the power transmission section 3 and is firmly fixed to the power transmission section 3, for example, by a key fit or an interference fit, so as to rotate the rotating body 111 by rotating the power transmission section 3. The rotating blades 112 are arranged on the outer side of the rotating body 111. A plurality of rotating-shaft-grooves 1111 are provided on the outer side of the rotating body 111. The rotating blades 112 undergone heat treatment, are removable from the rotating shaft grooves 1111, which allows the adjustment of the opening angle of the rotating blades 112 relative to the rotating body 111. Referring to FIG. 4, the rotating blades 112 are opened in three different angles: 0°, 45°, and 90°, respectively. So, the blood pump system has three operating states (as shown in FIG. 5): fully-folded, semi-unfolded, and fully-unfolded, to accommodate different applications. That is, the blood pump system can be delivered into the body through a common interventional surgery in the fully-folded state (which has the smallest profile), and then opened and switched to a semi-unfolded or fully-unfolded states within the body to enhance blood flow.

In addition, in one embodiment of the present disclosure, the outer side of the rotating body 111 is symmetrically provided with two sets of rotating shaft grooves 1111 along the axial direction of the rotating body 111. Each set of rotating shaft grooves includes three individual slots. The angle between each pair of adjacent rotating shaft grooves 1111 is 120 degrees. In other words, each set of rotating blades is situated adjacent to one set of rotating shaft grooves. The outer side of the rotating body 111 features two sets of rotating blades 112, comprising six rotating blades in total, to enhance the suction force generated by the rotation of the first rotating member 11. It can be understood that in one embodiment of the present disclosure, the aforementioned structure is only one specific implementation. In other embodiment, the number of the rotating shaft grooves and the number of the rotating blades can be adaptively adjusted as needed.

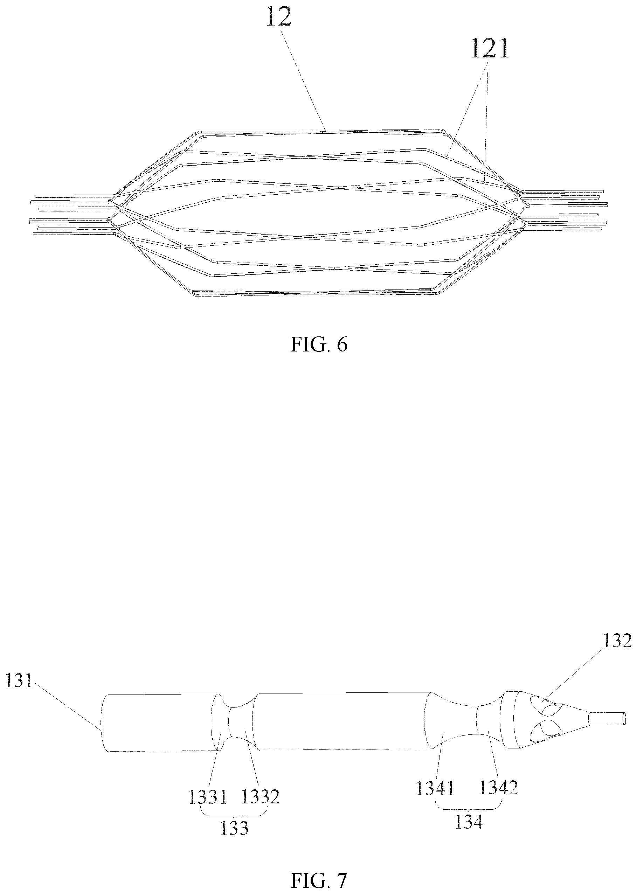

A first mesh stent 12 is installed on the outer side of the first rotating member 11, so as to protect the first rotating member 11 from hurting body tissues and improve the safety of using the heart circulation pump 1. Specifically, as shown in FIG. 6, in one embodiment of the present disclosure, the first mesh stent 12 is woven from multiple first metal wires 121, which can be made of ordinary metal or alloy. The middle section of each first metal wire is bent into a trapezoidal shape, so that both ends of the multiple first metal wires are sleeved on the power transmission section 3, and the middle section of the multiple first metal wires form a cylindrical cavity for accommodating and protecting the first rotating member 11.

The first guiding tube 13, is positioned on the outer side of the first mesh stent 12. The first guiding tube 13 has a first blood inlet 131 and a first blood outlet 132. The first blood inlet 131 is located at a distance from the aortic circulation pump 2. In contrast, the first blood outlet 132 is situated close to the aortic circulation pump 2. The first guiding tube 13 directs blood from the heart through the first blood inlet 131, into the tube, and then out of the tube through the first blood outlet 132. Specifically, as shown in FIG. 7, in one embodiment of the present disclosure, the first guiding tube 13 has a first acceleration section 133 and a second acceleration section 134. The first acceleration section 133 is a Coanda effect tube, also known as the wall effect or Coanda effect: the fluid (water flow or air flow) deviates from the original flow direction and tends to flow along the protruding surface of the object. The Coanda effect tube, the first acceleration section 133, specifically includes a first arc-shaped tube 1331 and a second arc-shaped tube 1332 with gradually changing diameters, both of which are bent towards an axis of the first guiding tube 13. The diameter of the first arc-shaped tube 1331 decreases from large to small, with a reduction value of ϕ99mm to ϕ0.1mm. The diameter of the second arc-shaped tube 1332 increases from small to large, with an expansion value of ϕ99mm to ϕ0.1mm, thus forming a Kanda effect area at the connection between the first arc-shaped tube 1331 and the second arc-shaped tube 1332 to accelerate the blood (the first acceleration).

Moreover, in one embodiment of the present disclosure, the second acceleration section 134 is a Venturi supersonic nozzle structure, commonly known as a supersonic nozzle or a critical flow nozzle, mainly used for transmitting flow standards, measuring gas flow rates, and limiting the maximum flow rate of a flow systems. Specifically, the second acceleration section 134 includes a third arc-shaped tube 1341 and a fourth arc-shaped tube 1342. Similarly, the third arc-shaped tube 1341 and the fourth arc-shaped tube 1342 both bend towards the axis of the first guiding tube 13. The diameter of the third curved tube 1341 decreases from large to small, with a reduction-value of ϕ99mm to ϕ0.1mm, and the diameter of the fourth curved tube 1342 increases from small to large, with an increase-value of ϕ99mm to ϕ0.1mm, thus forming a Venturi sonic nozzle action area at the connection between the third curved tube 1341 and the fourth curved tube 1342 to accelerate the blood (the second acceleration). As a result, the first guiding tube 13 can be used to double accelerate blood flow, thereby increasing blood flow velocity and assisting in improving cardiac blood supply function.

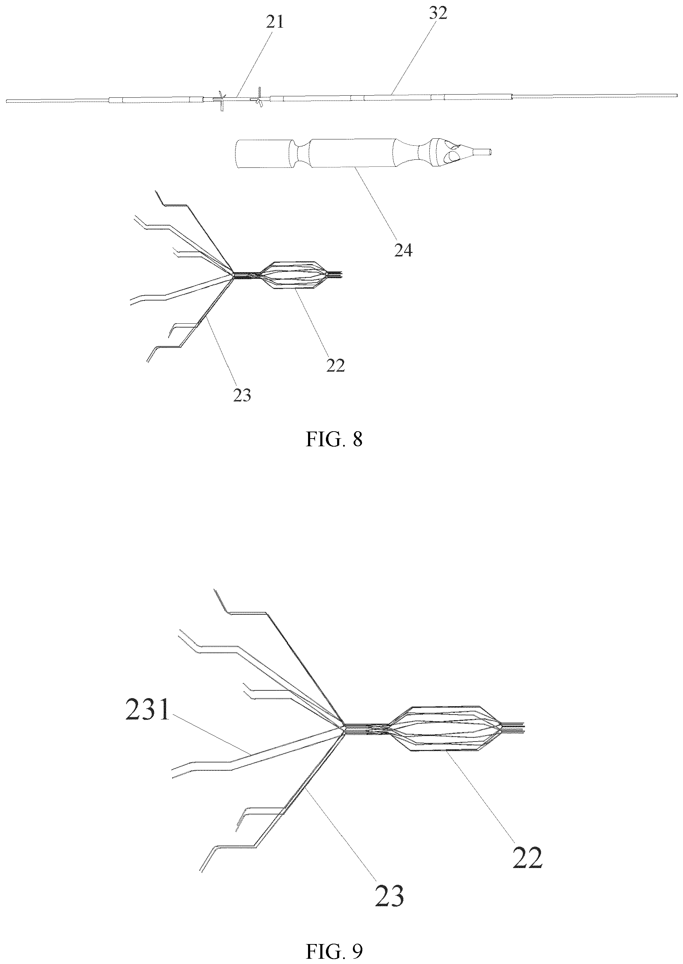

As shown in FIG. 8, in the above embodiment, the aortic circulation pump 2 includes a second rotating member 21, a second mesh stent 22, a supporting stent 23, and a second guiding tube 24. The second rotating member 21 is similar to the first rotating member 11, the second mesh stent 22 is similar to the first mesh stent 12, and the second guiding tube 24 is similar to the first guiding tube 13. Thus, they have the same structure as the heart circulation pump 1, and will not be repeated here. The difference between aortic circulation pump 2 and heart circulation pump 1 is that aortic circulation pump 2 further includes a supporting stent 23, as shown in FIG.9. The supporting stent 23 can spread outwardly around the power transmission section 3 in an open state so as to support the aorta. Specifically, the supporting stent 23 is woven with multiple second metal wires 231. Unlike in the second mesh stent 22, in the supporting stent 23, the first end of each second metal wire is close to the power transmission section 3, and the second end is far away from the power transmission section 3, so that the first ends of the multiple second metal wires are collectively sleeved on the power transmission section 3 and connects with the second mesh stent 22, and the second ends of the multiple second metal wires are spread in a petal shape. It can be understood that in one embodiment of the present disclosure, the second ends of multiple second metal wires are elastic, so that the second end of the supporting stent 23 can be contracted or spread. When it enters the aorta, the second end of the supporting stent 23 can be contracted to avoid interference. After entering the preset position of the aorta, the second end of the supporting stent 23, can be expanded to support the vascular wall of the aorta, which prevents blood flow from being blocked.

In addition, it can be understood that, in one embodiment of the present disclosure, the second mesh stent 22 and the supporting stent 23 can be integrated together. In another embodiment, they can also be designed as a two-piece structure, which allows for selection based on actual needs. In another embodiment, the first mesh stent 12 is laser engraved from a metal tube, so that both ends of the first mesh stent 12 are fitted onto the power transmission section 3, and the middle section of the first mesh stent 12 forms a cylindrical cavity for accommodating the first rotating member. And, the supporting stent 22 is formed by laser engraving from a metal tube, so that the first end of the supporting stent 22 is sleeved on the power transmission section 3, and the second end of the supporting stent 22 is spread out in a petal shape.

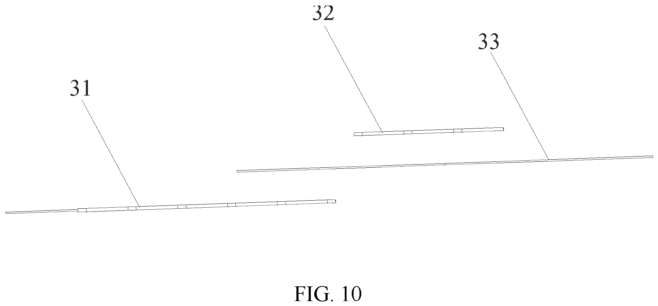

As shown in FIG. 10, in the above embodiment, the power transmission section 3 includes a cardiac transmission section 31, an aortic transmission section 32, and a power cable 33. The cardiac transmission section 31 can be directly connected to the power cable 33, so that the heart circulation pump 1 can be placed in the heart and work independently. The aortic transmission section 32 can be directly connected to the power cable 33, allowing the aortic circulation pump 2 to be placed inside the aorta and work independently. Alternatively, the cardiac transmission section 31 can be connected to the aortic transmission section 32, and then connected to the power cable 33 through the aortic transmission section 32, so that the heart circulation pump 1 can be placed in the heart and the aortic circulation pump 2 can be placed in the aorta, allowing both pumps to work simultaneously.

In summary, the blood pump system provided in the present embodiment can accelerate the circulations of blood in the heart and/or the aorta respectively through the heart circulation pump 1 and/or the aortic circulation pump 2, thereby assisting in improving the blood supply of the heart. Moreover, the rotating blades 112 of the blood pump system are adjustable. So, when in use, the blood pump system can be easily inserted into the body through a common interventional surgery in the fully-folded state (with the smallest profile), and then spread to a semi-unfolded or fully-unfolded state to increase blood flow and enhance convenience of use.

### Second Embodiment

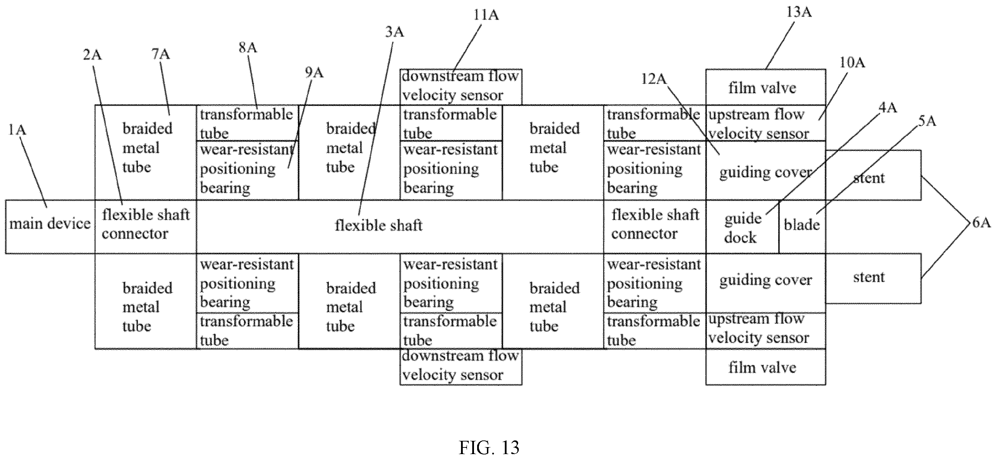

As shown in FIG. 13, a blood pump system is provided in the second embodiment of the present disclosure, which includes a main device 1A, multiple flexible shaft connectors 2A, multiple flexible shafts 3A, a guide dock 4A, a plurality of blades 5A, a stent 6A, multiple braided metal tubes 7A, multiple transformable tubes 8A, multiple wear-resistant positioning bearings 9A, an upstream flow velocity sensor 10A, a downstream flow velocity sensor 11A, a guiding cover 12A, and at least one film valve 13A.

Specifically, in one embodiment of the present disclosure, the main device 1A is located at the end of the blood pump system, and includes a power motor, a display, and a controller. The power motor is used to provide rotational power to the multiple flexible shafts 3A. The display is used for image display. The controller is used for program control of various sensors, valves, and blades. The monitor has multiple data interfaces that can read data from various sensors, valves, and blades. The above data interfaces include interfaces that comply with USB standards and can be used to output or import data. This controller can run software, which can analyze and calculate the appropriate blood flow rate at the current time based on the information collected through the data interface, and control the speed of blades 5A according to the calculation results. The main device 1A also includes one or more power source interfaces for connecting the flexible shaft 3A, and transmitting the power generated by the motor to the blade 5A through gears or other energy-delivery structures.

In one embodiment of the present disclosure, the blood pump system further comprises an operating handle for connecting the power source interface and data interface of the main device 1A, regulating the operations of the blades 5A and the guiding cover 12A. The material of the operating handle may be composed of ABS, nylon, polycarbonate, carbon fiber, fiberglass, etc.

The flexible shaft 3A is connected to the main equipment 1 through the flexible shaft connector 2A. And there is a flexible shaft connector 2A connected between adjacent two flexible shafts 3A. This flexible shaft connector 2A is used to connect with the main equipment 1A, thereby achieving the conversion between rigid transmission and flexible transmission. The flexible shaft 3A is composed of a single or multiple wires, which are made of stainless steel, carbon steel, titanium alloy, nickel titanium alloy or the like. The flexible shaft 3A is composed of multiple wires, with some metal wires woven together.

The guiding dock 4A is connected to the flexible shaft 3A through the flexible shaft connector 2A, and is equipped with the blades 5A at an end far from the main device 1A. The rotation of the blades 5A can accelerate blood to flow into the guiding dock 4A. Moreover, the guiding cover 12A is fixed on the outer side of the guiding dock 4A. The flow of blood accelerated by the blades 5A into the guiding dock 4A is ejected from the guiding cover 12A according to the design direction. Thus, a vortex is formed within the guiding cover 12A at one end thereof. The guiding cover 12A is used to accommodate the blades 5A to prevent collision between the blades 5A and body tissue, and to provide containment and restraint for blood flow. In addition, the guiding cover 12A may be composed of a thin film of polymer material, which can automatically expand and unfold, or it can expand under compression after the blades 5A rotates and sucks in fluid.

The blades 5A can be an integrated structure, a split structure, or composed of a woven metal skeleton with a surface membrane or coating. The material of the blades 5A can be organic or inorganic. The blades 5A can be extended or retracted, with the extended size ranging from 1mm to 3000mm, and the retracted size ranging from 1mm to 1000mm.

The guiding cover 12A can also be woven from metal wire or laser engraved from metal pipes, with elasticity or self-expansion, allowing for automatic unfolding. The metal materials that make up the guiding cover 12A may be titanium alloy, nickel titanium alloy, stainless steel, carbon fiber or polymer material sheets, or a combination of metal and polymer materials. The guiding cover 12A has protrusions distributed at one end where the blood flow in, which helps to achieve the Coanda Effect. By utilizing the Coanda Effect, when the blades 5A start rotating to suck in blood, the velocity and flow rate of blood flow inside the guiding cover 12A will increase, resulting in an increase in liquid pressure at the center section of the guiding cover 12A. This pathway inside the guiding cover 12A allows for faster and more efficient suction of blood flow than in a straight channel. The overall inside structure of the guiding cover 12A conforms to the Venturi effect (critical flow Venturi nozzle) property. Thus, when blood is drawn in from the inlet and accelerated by the blades 5A, it flows inside the Venturi tube. Due to the continuity equation, the velocity reaches its maximum value at the narrowest areas of the pathway, and the static pressure reaches its minimum value. Therefore, the speed of blood increases due to changes in the cross-sectional area of the pathway. The entire blood flow undergoes a narrowing process at the same time, resulting in a pressure difference that ensures a pressure loss of around 5% to 20% between the blood inlet and outlet.

The stent 6A is set at an end of the guiding cover 12A for fixing the guiding cover 12A to the blood vessel wall.

The plurality of metal tubes 7A are spaced around the outer side of the flexible shaft 3. Each transformable tube 8A is provided between adjacent metal tubes 7A. The inner layer of the metal tube 7A is braided with nickel titanium alloy wire, the outer layer is coated with hydrophilic polymer material. The outer layer is in contact with blood, the inner layer accommodates the soft shaft 3A. The soft shaft 3A is connected to the main equipment 1A at one end, and is connected to the blades 5A at the other end. The metal tube 7A is partially located outside the body (the end near the main device 1A), and the other end (the end near the blades 5A) is located inside the body. In addition, each of the transformable tubes 8A provides support to the metal tube 7A, and provides axial support and positioning for the soft shaft 2A through the internally fixed wear-resistant positioning bearing 9A.

The upstream flow velocity sensor 10A is mounted on the guiding cover 12A for detecting the blood flow velocity before acceleration. Similarly, the downstream flow velocity sensor 11A is installed in the central area of the blood pump system (for example, installed on one of the transformable tubes 8A or one of the metal tubes 7A in the central area) to detecting the accelerated blood flow velocity.

The film valve 13A are installed adjacent to the upstream flow velocity sensor 10A to control the outflow of blood that simulates the flow stimulated by heartbeats. This simulation is based on the electrocardiogram signals collected by the main device 1A. Consequently, the blood pump system is able to output blood flow in a pulse-like manner, similar to the output of the human heart. In one embodiment of the present disclosure, each outlet of the guiding cover 12A is equipped with one film valve 13A. The film valve 13A is driven by optokinetic energy, thermal energy, mechanical force, magnetic force, ultrasonic force, or electric force. The film valve 13A can be opened or closed according to the preset frequency and opening angle, or to the collected physiological signals that include electrocardiogram signals, temperature signals, flow velocity signals, or photoelectric signals.

### Third Embodiment

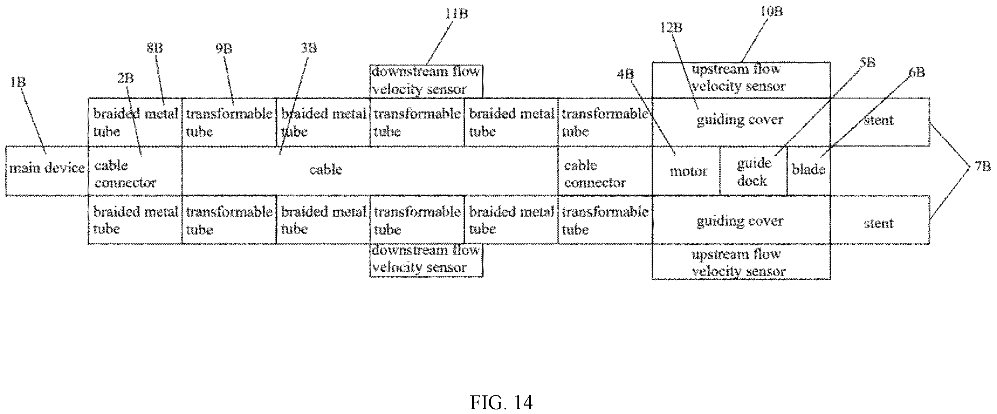

As shown in FIG. 14, a blood pump system provided in the third embodiment of the present disclosure includes a main device 1B, multiple cable connectors 2B, multiple cables 3B, a motor 4B, a guiding dock 5B, a plurality of blades 6B, a stent 7B, multiple braided metal tubes 8B, multiple transformable tubes 9B, an upstream flow velocity sensor 10B, a downstream flow velocity sensor 11B, and a guiding cover 12B.

Specifically, in one embodiment of the present disclosure, the main device 1B is located at the end of the blood pump system, and includes a power motor, a display, and a controller. The power motor is used to provide rotational power to the multiple flexible shafts 3A. The display is used for image display. The controller is used for program control of various sensors and blades. The monitor has multiple data interfaces that can read data from various sensors, valves, and blades. The above data interfaces include interfaces that comply with USB standards and can be used to output or import data. This controller can run software, which can analyze and calculate the appropriate blood flow rate at the current time based on the information collected through the data interface, and control the speed of blades 6B according to the calculation results. The main device 1B also includes one or more power source interfaces for connecting the cable 3B, and transmitting the power generated by the motor to the blade 6B through gears or other energy-delivery structures.

In one embodiment of the present disclosure, the blood pump system further comprises an operating handle for connecting the power source interface and data interface of the main device 1B, regulating the operations of the blades 5B and the guiding cover 12B. The material of the operating handle may be composed of ABS, nylon, polycarbonate, carbon fiber, fiberglass, etc.

The cable 3B is connected to the main equipment 1 through the cable connector 2B. And there is a cable connector 2B connected between adjacent two cables 3A. This cable connector 2B is used to connect with the main equipment 1A, thereby achieving the conversion between rigid transmission and flexible transmission. The cable 3B is connected to motor 4B of the blades 5B at one end, and the cable 3B is connected to the operating handle at another end.

The guiding dock 5B is connected to the cable 3B through the cable connector 2A, and is equipped with the blades 6B at an end. The rotation of the blades 6B can accelerate blood to flow into the guiding dock 5B. Moreover, the guiding cover 12A is fixed on the outer side of the guiding dock 5B. The flow of blood accelerated by the blades 6B into the guiding dock 5B is ejected from the guiding cover 12B according to the design direction. Thus, a vortex is formed within the guiding cover 12B at one end thereof. The guiding cover 12B is used to accommodate the blades 6B to prevent collision between the blades 6B and body tissue, and to provide containment and restraint for blood flow. In addition, the guiding cover 12B may be composed of a thin film of polymer material, which can automatically expand and unfold, and it can expand under compression after the blades 6B rotates and draws in fluid.

The blades 6B can be an integrated structure, a split structure, or composed of a braided metal skeleton with a surface membrane or coating. The material of the blades 6B can be organic or inorganic. The blades 6B can be extended or retracted, with the extended size ranging from 1mm to 3000mm, and the retracted size ranging from 1mm to 1000mm.

The guiding cover 12B can be braided from metal wire or laser engraved from metal pipes, with elasticity or self-expansion, allowing for automatic unfolding. The metal materials that make up the guiding cover 12B may be titanium alloy, nickel titanium alloy, stainless steel, carbon fiber or polymer material sheets, or a combination of metal and polymer materials. The guiding cover 12B has protrusions distributed at one end where the blood flows in, which helps to achieve the Coanda Effect. By utilizing the Coanda Effect, when the blades 6B start rotating to draw in blood, the velocity and flow rate of the blood flow inside the guiding cover 12B will increase, resulting in an increase in liquid pressure at the center of the guiding cover 12B. This pathway inside the guiding cover 12B allows for faster and more efficient suction of blood flow than in a straight pathway. The overall inside structure of the guiding cover 12B conforms to the Venturi effect (critical flow Venturi nozzle) property. Thus, when blood is drawn in from the suction end and accelerated by the blades 6B, it flows inside the Venturi tube. Due to the continuity equation, the velocity reaches its maximum value at the narrowest areas of the pathway, and the static pressure reaches its minimum value. Therefore, the speed of blood increases due to changes in the cross-sectional area of the pathway. The entire blood flow undergoes a narrowing process at the same time, resulting in a pressure difference that ensures a pressure loss of around 5% to 20% between the blood inlet and outlet.

The stent 7B is set at one end of the guiding cover 12B for fixing the guiding cover 12B to the blood vessel wall.

The plurality of metal tubes 8B are spaced around the outer side of the cable 3B. Each transformable tube 9B is provided between adjacent metal tubes 8B. The inner layer of the metal tube 8B is braided with nickel titanium alloy wire, the outer layer is coated with hydrophilic polymer material. The outer layer is in contact with blood, the inner layer accommodates the cable 3B. The cable 3B is connected to the main equipment 1A at one end, and is connected to the blades 5B at the other end. Some of the metal tubes 8B are located outside the body (near the main device 1A), and the others is located inside the body (near the blades 6B). In addition, each of the transformable tubes 9B provides support to the metal tube 8B, and provides axial support and positioning for the soft shaft 2B through the internally fixed wear-resistant positioning bearing 9B.

The upstream flow velocity sensor 10B is mounted on the guiding cover 12B for detecting the blood flow velocity before acceleration. Similarly, the downstream flow velocity sensor 11B is installed in the central area of the blood pump system (for example, installed on one of the transformable tubes 9B or one of the metal tubes 8B in the central area) to detecting the accelerated blood flow velocity.

The blood pump system provided by the present disclosure has been described in detail above. For general technical personnel in this field, any apparent alteration made on the present disclosure by persons of ordinary skilled in the art without departing from the substantive content of the present disclosure shall constitute an infringement of the patent right of the present disclosure, and the person shall bear corresponding legal liability.

## Claims

1. A blood pump system comprising:
a heart circulation pump, used to be set inside the heart to extract blood from the heart into the aorta;
an aortic circulation pump, used to be installed inside the aorta to supply blood to designated organs;
a power transmission section connected between the heart circulation pump and the aortic circulation pump to achieve power transmission.

2. The blood pump system according to claim 1, **characterized in that** the heart circulation pump comprises:
a first rotating member, mounted on the power transmission section to rotate with the power transmission section;
a first mesh stent, installed on an outer side of the first rotating member to protect to the first rotating member; and
a first guiding tube, sleeved on the outer side of the first mesh stent and including a first blood inlet and a first blood outlet, wherein the first blood inlet is far away from the aortic circulation pump and the first blood outlet is close to the aortic circulation pump,
the power transmission section configured to drive the first rotating member to rotate, so as to drive blood in a heart to enter the first guiding tube from the first blood inlet of the first guiding tube and then flow out of the first guiding tube from the first blood outlet.

3. The blood pump system according to claim 2, **characterized in that** the aortic circulation pump comprises:
a second rotating member, mounted on the power transmission section to rotate with the rotation of the power transmission section;
a second mesh stent, installed on an outer side of the second rotating member for protection to the second rotating member;
a supporting stent, being able to spread over the power transmission section in an open state to support an aorta; and
a second guiding tube, sleeved on an outer side of the second mesh stent and including a second blood inlet and a second blood outlet, wherein the second blood inlet is close to the first blood outlet and the second blood outlet is far away from the first blood outlet.

4. The blood pump system according to claim 2, wherein the first rotating member comprises:
a rotating body, sleeved on the power transmission section to rotate with the rotation of the power transmission section, and being opened with a plurality of rotating shaft grooves for accommodating the rotating blades;
a plurality of rotating blades, which can be accommodated in and extend from the rotating shaft grooves to adjust an opening angle of the rotating blades relative to the rotating body.

5. The blood pump system according to claim 2, **characterized in that** the first mesh stent is braided from a plurality of first metal wires, and a middle section of each first metal wire is bent into a trapezoidal shape, so that both ends of the first metal wires are sleeved on the power transmission section, and the middle section of the first metal wires forms a cylindrical cavity for accommodating the first rotating member.

6. The blood pump system according to claim 3, **characterized in that** the supporting stent is braided from multiple second metal wires, and a first end of each second metal wire is close to the power transmission section, and a second end of each second metal wire is far away from the power transmission section, so that the first ends of the second metal wires are collectively sleeved on the power transmission section, and the second ends of the second metal wires are spread in a petal shape.

7. The blood pump system according to claim 3, **characterized in that**:
the first mesh stent is laser engraved from a metal tube, so that both ends of the first mesh stent are fitted onto the power transmission section, and the middle section of the first mesh stent forms a cylindrical cavity for accommodating the first rotating member.

8. The blood pump system according to claim 3, **characterized in that**:
the supporting stent is laser engraved from a metal tube, so that a first end of the supporting stent is sleeved on the power transmission section, and a second end of the supporting stent is spread out in a petal shape.

9. The blood pump system according to claim 3, **characterized in that** the power transmission section comprises a cardiac transmission section, an aortic transmission section, and a power cable;
the cardiac transmission section is directly connected to the power cable to control the rotation of the first rotating member through the power cable;
the aortic transmission section is directly connected to the power cable to control the rotation of the second rotating member through the power cable;
the cardiac transmission section is connected to the aortic transmission section, which is connected to the power cable to control the simultaneous rotation of the first rotating member and the second rotating member through the power cable.

10. The blood pump system according to claim 2, wherein the first guiding tube comprises a first acceleration section and a second acceleration section, which are spaced along the length direction of the first guiding tube to increase flow velocity of blood inside the first guiding tube.

11. The blood pump system according to claim 10, **characterized in that** the first acceleration section comprises a first arc-shaped tube and a second arc-shaped tube with gradually changing diameters, and both the first arc-shaped tube and the second arc-shaped tube are bent towards the axis direction of the first guiding tube;
the diameter of the first arc-shaped tube decreases, and the diameter of the second arc-shaped tube increases;
the bending radius of the first arc-shaped tube has a first set ratio to the bending radius of the second arc-shaped tube.

12. The blood pump system according to claim 10, **characterized in that** the second acceleration section comprises a third arc-shaped tube and a fourth arc-shaped tube, both of which are bent towards the axis direction of the first guiding tube; the diameter of the third arc-shaped tube decreases, and the diameter of the fourth arc-shaped tube increases; the bending radius of the third arc-shaped tube has a second set ratio to the bending radius of the fourth arc-shaped tube.
